# EUROPEAN PATENT SPECIFICATION

(11) **EP 3 712 123 B1**
(45) Date of publication and mention of the grant of the patent: **11.05.2022**
(21) Application number: 19164412.9
(22) Date of filing: 21.03.2019
(51) Int. Cl.: C07B 59/00, C07C 303/06, C07C 309/04

(54) **PROCESS FOR THE PREPARATION OF ISOTOPE LABELLED ALKANE SULFONIC ACID**
VERFAHREN ZUR HERSTELLUNG VON ISOTOPENMARKIERTER ALKANSULFONSÄURE
PROCÉDÉ DE PRÉPARATION D'ACIDE SULFONIQUE D'ALCANE MARQUÉ PAR ISOTOPE

(43) Date of publication of application: 23.09.2020
(73) Proprietor: Grillo-Werke AG, 47169 Duisburg (DE)
(72) Inventor: OTT, Timo, 47169 Duisburg (DE)
(74) Representative: dompatent von Kreisler Selting Werner - Partnerschaft von Patent- und Rechtsanwälten mbB

(56) References cited:
- WO-A1-2015/071365
- WO-A1-2018/096138
- BURLINGHAM B T ET AL: "Synthesis and Reactivity of Polydisulfonimides", JOURNAL OF THE AMERICAN CHEMICAL SOCIETY, AMERICAN CHEMICAL SOCIETY, US, vol. 123, no. 13, 1 January 2001 (2001-01-01), pages 2937-2945, XP002410818, ISSN: 0002-7863, DOI: 10.1021/JA002465V

## Description

The present invention relates to a process for preparing isotope labelled alkane sulfonic acids from sulfur trioxide and an alkane by adding a suitable initiator or initiator precursor, said initiator or initiator precursor provides the isotopes for the isotope labelled alkane sulfonic acid.

The existence of isotopes was first suggested by Frederick Soddy in the early 20^{th} century. This was based on studies of radioactive decay chains. Today, not only radioactive but also stable isotopes of several atoms are known. Usually, a neutral atom has the same number of electrons as protons. This does not change for isotopes. Therefore, different isotopes of one atom usually show the same or at least similar chemical behaviour. No rule without exception: the kinetic isotope effect is well known especially for hydrogen and the isotopes ¹H (protium), ²H (deuterium), and ³H (tritium). Isotopes with larger masses react somewhat more slowly than lighter isotopes of the same element. As the mass difference between protium, deuterium, and tritium is quite large, the chemical behaviour is different.

Isotopes of chemical elements are nowadays used e.g., for isotope analysis in the determination of isotopic signature. Isotopic substitution can also be used to determine the mechanism of a chemical reaction vie the kinetic isotope effect. Especially Radionuclides have important uses in nuclear medicine and radiation oncology for example. Especially in the medical area, it is of interest to provide isotopes in a form acceptable for the human or animal body. Medicaments are in principle known, but there is the need to provide them with isotopes for specific uses. Thus, there is a need for compounds enabling the synthesis of other isotope labelled molecules with more complex structures.

J. Am. Chem. Soc. (2001) 123, 2937-2945 discloses in Scheme 9 the preparation of deuterated methanesulfonic acid sodium salt from deuterated methyliodide. WO2018/096138 discloses a solvent-free alkane sulfonation.

Surprisingly it was found, that alkane sulfonic acids or intermediates in their preparation are suitable compounds for the functionalization of compounds and thus, to introduce isotopes into molecules. The inventor of the present application now found a cost effective and fast method to provide isotope labelled alkane sulfonic acids, especially methane sulfonic acid, which can be used to introduce different kinds of isotopes into molecules or may be used as such.

In a first aspect, the present application refers to a method for preparing isotope labelled alkane sulfonic acid, especially methane sulfonic acid, comprising the following steps:
i) providing an initiator or initiator precursor,
ii) providing SO₃ and
iii) providing the alkane,
iv) reacting initiator precursor, SO₃ and alkane in a reactor under a pressure of 1 bar up to 200 bar, and thereby controlling the temperature to be within a range of from 0 °C to 100 °C,
v) separating the thus obtained labelled alkane sulfonic acid from non-reacted educts and/or where appropriate side products,
   characterized in that
   the obtained alkane sulfonic acid is labelled with at least one isotope selected from ²H (D), ³H (T), ¹¹C, ¹³C,¹⁴C, ¹⁷O, ¹⁸O, ³³S, ³⁴S,³⁵S, ³⁶S,³⁸S as well of mixtures of two or more of them, wherein the concentration of the isotope is at least twice the amount of the natural amount of the respective isotope,
   wherein
      - the hydrogen isotope(s) in the alkane sulfonic acid are provided by using D₂SO₄ and T₂SO₄ respectively as solvent of the reaction and/or to provide SO₃ as oleum and/or
      - the C isotope(s) in the alkane sulfonic acid is provided by using the corresponding C-labelled alkane as educt and/or
      - the oxygen isotope(s) are provided by using the correspondingly labelled SO₃ as well as the correspondingly labelled H₂SO₄ as educt and/or solvent,
         and/or

      - the sulfur isotope(s) are provided by using the correspondingly labelled SO₃ as well as the correspondingly labelled H₂SO₄ as educt and/or solvent.

Surprisingly it was found, that the method of the invention enables the production of pure isotope labelled alkane sulfonic acid. The amount of isotopes is dependent on the amount of the respective isotope in the educt.

In one preferred embodiment, the method for providing an isotope labelled alkane sulfonic acid comprises the following steps:
a. Providing sulfur trioxide SO₃;
b. Providing an alkane, especially methane;
c. Providing a solvent,
d. Bringing into contact SO₃, alkane and the solvent in a high-pressure autoclave or laboratory reactor;
e. Setting a pressure of from 1 to 200 bar;
f. Adding an initiator or initiator precursor which is able to initialize the reaction between SO₃ and alkane at the described reaction conditions;
g. Controlling the temperature of the reaction mixture at 0 °C to 100 °C; letting react the compounds so that the alkane sulfonic acid, especially methane sulfonic acid, is formed;
   characterized in that
   the obtained alkane sulfonic acid is labelled with at least one isotope selected from ²H (D), ³H (T), ¹¹C, ¹³C,¹⁴C, ¹⁷O, ¹⁸O, ³³S, ³⁴S,³⁵S, ³⁶S,³⁸S as well of mixtures of two or more of them, wherein the concentration of the isotope is at least twice the amount of the natural amount of the respective isotope,
   wherein
      - the hydrogen isotope(s) in the alkane sulfonic acid are provided by using D₂SO₄ and T₂SO₄ respectively as solvent of the reaction and/or to provide SO₃ as oleum and/or
      - the C isotope(s) in the alkane sulfonic acid is provided by using the corresponding C-labelled alkane as educt and/or
      - the oxygen isotope(s) are provided by using the correspondingly labelled SO₃ as well as the correspondingly labelled H₂SO₄ as educt and/or solvent,
         and/or
      - the sulfur isotope(s) are provided by using the correspondingly labelled SO₃ as well as the correspondingly labelled H₂SO₄ as educt and/or solvent.

The solvent is preferably sulfuric acid, especially isotope labelled sulfuric acid, i.e. D₂SO₄ and/or T₂SO₄.

The method of the present invention is suitable to produce different kinds of isotope labelled alkane sulfonic acids. Preferably, the alkane which is used as educt and from which the respective alkane sulfonic acid is formed, is a short chain methane with 1 to 10 C atoms which can be linear or non-linear. Preferably, the alkane is selected from ethane, methane, propane, butane, and pentane. Preferably, the alkane is methane so that the isotope labelled alkane sulfonic acid being formed according to the method of the present invention is isotope labelled methane sulfonic acid.

Each atom of the alkane sulfonic acid, especially methane sulfonic acid, can be an isotope within the meaning of the present invention. It is also possible to provide the alkane sulfonic acid with more than one, namely two, three or four isotopes within the molecule. Thus, it is possible to provide alkane sulfonic acid, in which substantially all hydrogen atoms of the alkane are replaced by deuterium and/or tritium. It is also within the meaning of the present invention that the alkane sulfonic acid comprises for example hydrogen-isotope as well as carbon isotope(s). Of course, also hydrogen and sulphur, or hydrogen and oxygen, or oxygen and sulphur, or sulphur and carbon. Also, three types of atoms can isotopically be labelled within the meaning of the present invention. If methane sulfonic acid is produced with the method according to the present invention, it is possible to provide for example C^{x}H₃SO₃H, ^{x}CH₃SO₃H, CH₃^{x}SO₃H, ^{x}C^{x}H₃SO₃H, ^{x}CH₃S^{x}O₃H, and so on, with ^{x} meaning that the following atom is an isotope of the respective atom.

The method of the present invention enables to provide isotope labelled alkane sulfonic acids, especially methane sulfonic acid, wherein the concentration of the isotope or each of the isotopes in the molecule is at least twice the amount of the natural amount of the respective isotope. In a preferred embodiment, the concentration is at least three times the amount of the natural amount of the respective isotope.

Relevant to introduce the isotopes into the alkane sulfonic acid is to choose the respective educt. Isotopic labelled sulphuric acid can be obtained by dissolving SO₃ in D₂O or T₂O. Thus, D₂SO₄ and T₂SO₄ can easily be obtained. Isotopes of sulphur can be used to obtain sulphuric acid or SO₃ with S-iostopes. The sulphur can be oxidized to obtain the needed educts. Most of the isotopes which can be used according to the present invention are stable isotopes. But the method of the present invention is also fast enough to also provide alkane sulphonic acids, especially methane sulphonic acid, with radioactive isotopes for example. After the reaction, the amount of the isotope is still high enough to be used in different kind of applications, i.e. in the medicinal sector or for detecting structures etc.

The reaction between SO₃ and the alkane in the present solvent usually takes place in a high-pressure autoclave. Thus, the pressure at which SO₃ and alkane are contacted with each other is preferably in a range of from 1 bar to 200 bar, especially from 50 bar to 150 bar, preferably from 80 bar to 120 bar.

The temperature during the reaction is preferably within a range of from 0 °C to 100° C, especially from 15 °C to 80° C, especially preferred from 20 °C to 70° C, preferably from 35 °C to 60° C. Surprisingly, it was found that that formation of solid products is lower at low temperatures. Therefore, in a preferred embodiment, the temperature at which SO₃ and alkane are contacted with each other is below 70 °C, especially below 65 °C, preferably below 60 °C and especially preferred below 55 °C. If the temperature is around 0 °C or 10 °C, the reaction time increases tremendously so that for an economically process the temperature is preferably 20 °C or above, especially 25 °C or above, especially preferred 30 °C or above.

The SO₃ may be provided in a gaseous state (pure SO₃). Alternatively, it is possible to provide SO₃ as oleum, i.e. dissolved in H₂SO₄. In a preferred embodiment, sulfur trioxide is used in a form of oleum with a trioxide content of 50 % (w/w) or less, or 65 % (w/w) or more. Surprisingly it has been found that contrary to the prior art for the processes of the present invention also oleum with a sulfur trioxide content of 65 % (w/w) or more, especially of 70 % w/w or more can be used without negatively affecting the inventive process. Even pure sulfur trioxide (100 % (w/w) sulfur trioxide) may be used. Surprisingly it has been found that instead of using oleum, as described in the prior art, also pure sulfur trioxide can be used according to the present invention. This avoids the preparation of sulfur trioxide solutions. The reaction conditions are here without added solvents. Further, non-reacted sulfur trioxide can evaporate, avoiding the necessity quenching it.

The initiator or initiator precursor added at step f) of the preferred method according to the above preferred embodiment may be a compound being known to initialize the reaction between an alkane and SO₃ to form alkane sulfonic acid. Therefore, the compound added at step f) is preferably selected from the group consisting of organic or inorganic peroxides being stable at room temperature, compounds with a heterogeneously or homogenously cleavable bond, stable cations as well as mixtures of two or more of them. Suitable compounds are for example disclosed in WO2018/096138 EP 18157127.4, EP 18196493.3, EP 18196498.2, and EP 18196520.3, EP 18196520.3. In the following preferred compounds which can be used as initiator or initiator precursor are described. The terms initiator and catalyst are used synonym in the following.

Especially, preferred the initiator or initiator precursor is a compound according to the following formula (I):

ALK-SO₂-O-O-X (I)

wherein ALK is a branched or unbranched alkyl group, especially a methyl, ethyl, propyl, butyl, isopropyl, isobutyl group, or a higher alkyl group, and X = hydrogen, zinc, aluminium, an alkali or alkaline earth metal. Higher alkyl group within the meaning of the present invention means an alkyl group with 1 to 10 carbon atoms. Preferably, ALK is methyl, ethyl, propyl, butyl, isopropyl or isobutyl, especially methyl or ethyl, particularly methyl. X is preferably hydrogen, alkali or alkaline earth metal. Particularly, X is hydrogen. Thus, in a very preferred embodiment, the compound added to initialize the reaction between SO₃ and alkane is CH₃-SO₂-O-O-H.

Alternatively, the initiator or initiator-precursor added in step f) of the preferred embodiment is an organic peroxoacid which, where appropriate, comprises functional groups. In general, the peroxoacid according to the invention can be described by the formula R-O-O-H. Without the intention of being bound by theory, it is assumed that the peroxoacid acts by activating sulfur trioxide towards the reaction with an alkane. In a preferred embodiment the peroxoacid comprises at least one organic peroxoacid of sulfur, phosphorus, silicon, boron, nitrogen or carbon. Any suitable peroxoacid of said elements can be used. The peroxoacids are typically derived from the corresponding oxoacid of the respective element.

Preferably, the peroxoacid used as catalyst according to the invention comprises a peroxoacid group corresponding to -E(=X)ₘ(-YZ)ₙ-O-O-Z, wherein E is selected from the group consisting of S, P, Si, B, N and C, wherein X and Y may be the same or different and are selected from the group consisting of O and S, wherein m is an integer of from 0 to 2, wherein n is an integer of from 0 to 2, and wherein Z is H, Li, Na and/or K.

In a preferred embodiment of the invention, the peroxoacid group is selected from the group consisting of -SO₂-O-O-X, -CO-O-O-X, -PO(OH)-O-O-X, PS(OH)-O-O-X, wherein X is H, Li, Na and/or K. Surprisingly, it has been found that said preferred peroxoacids are particularly suitable as catalyst in the preparation of alkanesulfonic acids from alkanes and sulfur trioxide.

According to the invention, the organic peroxoacid comprises at least one additional functional group. The additional functional group may particularly be selected from the group consisting of carbon double bonds, carbon triple bonds, aryl groups, heteroaryl groups and functional groups comprising heteroatoms, especially functional groups comprising O, S, N, P, Si, B, Se, Te, F, CI, Br, I, Mg or Li atoms.

Particularly preferred are aryl groups, halogen atoms, such as F, CI, Br, I, and siloxane groups. The functional groups, particularly aryl groups, may be further derivatized and may contain further functional groups. Examples of functional groups according to the invention comprise particularly phenyl groups, carbonyl groups, ether groups, thioether groups, thioketone groups and halide groups.

Examples of suitable organic peroxoacids according to the invention are peroxybenzoic acid and trifluoroperacetic acid. Any of the aforementioned examples may be derivatized and/or substituted with side chains, particularly with alkyl groups, aryl groups or halogen atoms.

The organic peroxoacid used as an initiator according to the invention may be obtainable by a reaction of the corresponding oxoacid with a peroxide. More preferably, the peroxoacid may be obtainable by a reaction of the corresponding oxoacid with hydrogen peroxide or a salt thereof. Without the intention of being bound by theory, the reaction of an oxoacid with hydrogen peroxide can for example be described by

-EOₓ(OH)_{y}-OH + H₂O₂ -> -EOₓ(OH)_{y}-O-OH + H₂O. (R3)

If such an organic peroxoacid is used as compound, it is only suitable if it does not give rise to superacid conditions in the reactor at which the reaction takes place. This may be controlled by selecting the compound carefully or by using it in only small amounts.

In a further preferred embodiment, the compound is an inorganic peroxoacid or a salt thereof, wherein the peroxoacid is stable at room temperature. Stability at room temperature is particularly to be understood as stability in a reaction solvent comprising sulfur trioxide and an alkane, especially methane. This solvent may be sulfuric acid. The peroxoacid according to the invention must be stable enough in order to act as catalyst in the production of alkanesulfonic acids and not to decompose. Said decomposition may particularly take place by the release of reactive oxygen species such as superoxide anions (O₂⁻). In this sense, stability of the peroxoacid catalysts of the present invention for example means the absence of the release of reactive oxygen species such as superoxide anions.

In a preferred embodiment the peroxoacid comprises at least one peroxoacid of boron, silicon, phosphorus, carbon, nitrogen or sulfur. Any suitable peroxoacid of said elements can be used. The peroxoacids are typically derived from the corresponding oxoacid of the respective element.

Preferably, the peroxoacid used as a catalyst according to the invention is obtainable by a reaction of the corresponding oxoacid with a peroxide. More preferably, the peroxoacid is obtainable by a reaction of the corresponding oxoacid with hydrogen peroxide. Without the intention of being bound by theory, the reaction of an oxoacid with hydrogen peroxide can for example be described by

EOₓ(OH)_{y}-OH + H₂O₂ -> EOₓ(OH)_{y}-O-OH + H₂O. (R3)

In a preferred embodiment, the peroxoacid used according to the invention comprises a polyprotic acid. Particularly, the peroxoacid may consist of one or more polyprotic acids. Said polyprotic peroxoacid comprises one or more peroxy groups, which can be described by -O-O-X, wherein X may be hydrogen and/or an alkaline and/or alkaline-earth metal. More preferably X is hydrogen, lithium, sodium and/or potassium. Most preferably, X is hydrogen.

Preferably, if a polyprotic acid is used, the peroxoacid comprises one or more hydroxyl groups in addition to the one or more peroxy groups. Said hydroxyl groups may be present in form of a salt, i.e., the groups can be described by - O-X, wherein X may be hydrogen, an alkaline metal and/or an alkaline-earth metal. Most preferably X is hydrogen. The replacement of hydrogen with an alkaline-(earth) metal, however, may be particularly necessary to stabilize the peroxoacid as required by the invention.

In a preferred embodiment of the invention, the reaction product of phosphoric acid (H₃PO₄) with hydrogen peroxide, the reaction product of boric acid (H₃BO₃) with hydrogen peroxide and/or potassium peroxomonosulfate (KHSO₅) is used as stable inorganic peroxoacid according to the invention. Surprisingly, it has been found that said preferred peroxoacids are particularly suitable as catalyst in the preparation of alkanesulfonic acids from alkanes and sulfur trioxide.

If such an inorganic peroxoacid is used as compound, it is only suitable if it does not give rise to superacid conditions in the reactor at which the reaction takes place. This may be controlled by selecting the compound carefully or by using it in only small amounts.

In another embodiment, the compound is a compound comprising a heterolytically cleavable bond between an atom selected from the group consisting of nitrogen, phosphor sulfon oxygen and an atom selected from the group consisting of nitrogen, phosphor and sulfur.

A heterolytically cleavable bond in the sense of the present invention is especially a chemical bond -X-Y- between two atoms X and Y, which may be broken in such a way that the remaining fragments are not two radicals with unpaired electrons. Particularly, the electrons of the bond are unequally partitioned between atoms X and Y upon cleavage of the bond. The atoms X and Y of the heterolytically cleavable bond may additionally be bound to the same or different radicals. The bond between X and Y may be polarized. Polarization of the bond may enable or favor heterolytical cleavage of the bond. Polarization may, for example, be accomplished by choosing two different elements for atoms X and Y, especially elements with different electronegativities. Polarization of the bond may also be accomplished by choosing different radicals, to which atoms X and Y are additionally bound. These measures may be combined, when X and Y are different and bound to at least two different additional radicals. In principal, any compound comprising heterolytically cleavable bonds in the aforementioned sense can be employed according to the invention. Such compounds are cheaply available from commercial distributors.

In a preferred embodiment the heterolytically cleavable bond is a single bond or a double bond. Alternatively, the heterolytically cleavable bond may also be a triple bond. Particularly preferred are single bonds.

The bond is preferably heterolytically cleavable under acidic conditions. Particurly preferred are bonds, which are heterolytically cleavable under superacid conditions. Alternatively, the bond may be heterolytically cleavable at a pH value of 3 or less, preferably of 0 or less. The pH value is preferably at least -10.

Heterolytic cleavage of the bond of the inventive catalyst preferably generates a cation and/or an anion. If the cleavage of the bond is catalyzed by an acid, particularly H⁺, the anion may formally react with the acid upon cleavage. In this case, only a cation and a neutral compound are generated.

The compound, which is used as catalyst according to the invention, is preferably selected from the group consisting of R¹-N-N-R², R¹-N-O-R², R¹-N-P-R², R¹-S-N-R², R¹-P-P-R², R¹-P-O-R², R¹-S-P-R², R¹-S-O-R² and R¹-S-S-R², wherein R¹ and R² may be the same or different and are selected from the group consisting of hydrogen, organic radicals and inorganic radicals. Particularly if the catalyst is chosen from R¹-N-N-R², R¹-P-P-R² and R¹-S-S-R¹, R¹ and R² may be different in order to polarize the bond and favor heterolytic cleavage of the bond.

In a preferred embodiment R¹ and/or R² are selected from the group consisting of -E(=X)ₘ(-YZ)ₙ, -E(=X)ₘ(-YZ)ₙ-R³, -E(=X)ₘ(-YZ)ₙ-R³R⁴ and-E(=X)ₘ(-YZ)ₙ-R³R⁴R⁵, wherein R³, R⁴ and R⁵ are each directly bonded to E, may be the same or different and are selected from the group consisting of hydrogen, organic radicals and inorganic radicals, wherein E is selected from the group consisting of S, P, Si, B, N and C, wherein X and Y may be the same or different and are selected from the group consisting of O and S, wherein m is an integer of from 0 to 2, wherein n is an integer of from 0 to 3 and wherein Z is H, Li, Na and/or K.

In these preferred embodiments, the atom X and/or the atom Y, which form the heterolytically cleavable bond, may each be directly bound to an atom E selected from S, P, Si, B, N and C. Depending on the valence of atom E, said atom may additionally be bound to up to three further radicals R³, R⁴ and R⁵. The place of the radicals R³, R⁴ and R⁵ can alternatively be filled with oxygen or sulphur atoms, which are double bonded to E, or groups YZ, which primarly correspond to OH and SH groups and their derivates. The inventive catalyst compound may therefore, for example, be a derivate of an oxoacid of sulfur, phosphorus, silicon, boron, nitrogen or carbon.

In a particularly preferred embodiment of the invention, R¹ and/or R² are selected from the group consisting of -SO₂-R³, -SO₂OH, -CO-R³, -PO(OH)-R³, -PS(OH)-R³, Si(OH)₃, -Si(OH)₂-R³, SiH₃ and -SiH₂-R³. Surprisingly, it has been found that such compounds are particularly suitable as catalyst in the preparation of alkanesulfonic acids from alkanes and sulfur trioxide.

Each of the aforementioned radicals R¹, R², R³, R⁴ and R⁵ may preferably be individually selected from the group consisting of alkyl radicals, alkyl radicals substituted with one or more functional groups, siloxane radicals or any other suitable inorganic or organic radical.

Preferred alkyl radicals are branched or unbranched alkyl radicals with a carbon number of 1 to 20, especially 1 to 10, particularly methyl, ethyl, propyl, butyl, isopropyl, isobutyl or higher alkyl radicals.

The aforementioned additional functional groups may particularly be selected from the group consisting of carbon double bonds, carbon triple bonds, aryl groups, heteroaryl groups and functional groups comprising heteroatoms, especially functional groups comprising O, S, N, P, Si, B, Se, Te, F, CI, Br, I, Mg or Li atoms.

Particularly preferred are aryl groups, halogen atoms, such as F, CI, Br, I, and siloxane groups. The functional groups, particularly aryl groups, may be further derivatized and may contain further functional groups. Examples of functional groups according to the invention comprise particularly phenyl groups, carbonyl groups, ether groups, thioether groups, thioketone groups and halide groups.

In a preferred embodiment, the compound according to the invention is part of or bound to an organic or inorganic polymer. Any suitable polymer may be chosen. Particularly preferred polymers comprise polysiloxanes, polyolefins, vinyl polymers, polyether, polyester, polyamides and polyurethanes. The catalyst compound group may be bound to the polymeric backbone or may be contained in a polymeric side chain.

The polymer may have any suitable structure. Particularly, homopolymers, copolymers, block copolymers, graft copolymers or comb copolymers may be employed. The polymers may have a dendrimer structure.

In yet another preferred embodiment, the initiator or initiator precursor added in step f) of the preferred method is a cation being stable under super acid conditions. If the cation is stable, stability in this context means that it is able to react with the alkane but does not decompose within 24 h at room temperature (20 °C), i.e. the half-life time t_{1/2} at room temperature is at least 24 h, preferably at least 30 h, especially at least 48 h.

Stable cations are formed prior to their use, i.e. prior to their addition into the reactor in which the reaction between alkane and sulfur trioxide takes place. Preferably, one type of cation is used alone and not together with another type of cation.

Alternatively, the cation is produced *in situ* during the production of the alkane sulfonic acid. In such cases a compound is added to the reaction and the cation is formed according to the above shown reaction (R2). Suitable compounds to be used are halogens, especially I₂ and Br₂, inter halogen compounds, especially I-Br, or solid elements of the 15^{th} or 16^{th} group of the periodic table of elements, especially S, Se, Te, P, As, Sb.

If halogens or interhalogens are used as compounds, the bond between the halogens breaks heterolytically. Iodine would thus react to HI and I⁺, bromine to HBr and Br⁺, and the interhalogen either to HI and Br⁺ or to HBr and I⁺. The reactive cation would be I⁺ or Br⁺, which is formed *in situ,* and afterwards reacts with the alkane to the alkane cation as schematically shown above in (R1).

If solid elements of the 15^{th} or 16^{th} group of the periodic table are used, they may form oligomeric or polymeric cationic compounds, i.e., sulfur will form an oligomer Sₙ-S⁺, wherein n may for example be in the range of from 0 to 10, preferably from 2 to 10, or in another range. Said Sₙ-S⁺ would be the reactive compound. Similar compounds may also occur with the other elements. Alternatively, they can form cations without polymerisation/oligomerisation. To sum up all possible cations of S, Se, Te, As, Sb and P, they are summarized with S⁺, Se⁺, Te⁺, As⁺, Sb⁺, and P⁺ respectively. Additionally, also Silicon may be added to the reaction solution forming Si⁺ as cation.

The method of the present invention enables an effective way to obtain isotope labelled alkane sulfonic acid, especially methane sulfonic acid. This acid obtained from the inventive method can be used to introduce isotopes into organic molecules. These organic molecules may be suitable to be used as medicament for humans or animals. Alternatively, the labelled acid may be further processed and afterwards used to label an organic compound, such that the alkane sulfonic acid residue ALK-SO₃- is attached to the molecule.

With the following non limiting examples, the present invention is shown in preferred embodiments.

### Examples:

### Example 1:

Deuterated oleum (SO₃/D₂SO₄) was prepared by slow dropwise addition of SO₃ into an ice bath cooled solution of 95-98% D₂SO₄ to afford a concentration of 33% SO₃. The temperature of the mixture must be carefully controlled to avoid overheating. The freshly prepared SO₃/D₂SO₄ (267.49 g, 1.105 mol SO₃) was added into a 400 mL stainless steel high-pressure reactor equipped with two oblong quartz windows using an HPLC pump heated to 50 °C. The reactor was previously cleaned, dried and purged with nitrogen. The reactor was heated to 50 °C and was filled with 96.4 bar of CD₄. The electrophilic initiator was prepared by dropwise addition of D₂O₂ 60% (464 µL, 9.74 mmol) over a cooled (0 °C) mixture of D-MSA 99.5% (1.38 mL, 21.25 mmol) in D₂SO₄ 95-98% (12 mL, 212 mmol). Once the temperature and pressure remained stable, the electrophilic initiator was added using an HPLC pump into the reactor. After 16 h, the pressure dropped to 31.8 bar, indicating that a large amount of methane was consumed. The reactor was then cooled down to room temperature, the excess pressure was removed to a set of scrubbers and the product consisting of a slightly colorless liquid was stored in a glass bottle. The sample was analyzed using ion chromatography affording 63,2% yield and 99.8% selectivity of D-MSA based on the total initial moles of SO₃.

D-MSA: CD₃SO₃H

### Example 2:

The electrolysis of pure D-MSA according to WO 2015/071371 A1 with platinum electrodes yields to d6-DMSP in very good yields. d6-DMSP can be used to synthesis deuterated methanesulfonic esters.

Syntheses of d3-Methansulfonic phenylester:
To 20 mL of a solution of d6-DMSP in d3-MSA - directly taken from the electrolysis-cell - an excess of benzene was added. The solution was stirred extensively and heated up to 60°C. After 60 minutes the reaction was finished. Unreacted benzene was distilled off under vacuum. The resulting mixture got diluted with 50% distilled water and extracted with Dichloromethane. The extract was purified with coloumn-chromatographie with silica gel. The pure product was obtained in 67% yield after evaporation of the solvent.

The following reaction takes place:

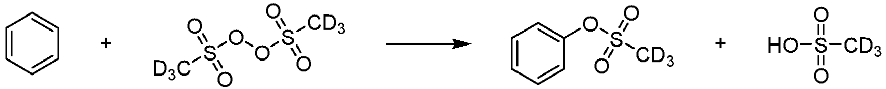

## Claims

1. Method for preparing isotope labelled alkane sulfonic acid, especially methane sulfonic acid, comprising the following steps:
i) providing an initiator precursor,
ii) providing SO₃ and
iii) providing the alkane,
iv) reacting initiator precursor, SO₃ and alkane in a reactor under a pressure of 1 bar up to 200 bar, and thereby controlling the temperature to be within a range of from 0 °C to 100 °C,
v) separating the thus obtained labelled alkane sulfonic acid from non-reacted educts and/or where appropriate side products,
**characterized in that**
the obtained alkane sulfonic acid is labelled with at least one isotope selected from ²H (D), ³H (T), ¹¹C, ¹³C,¹⁴C, ¹⁷O, ¹⁸O, ³³S, ³⁴S,³⁵S, ³⁶S,³⁸S as well of mixtures of two or more of them, wherein the concentration of the isotope is at least twice the amount of the natural amount of the respective isotope,
wherein
- the hydrogen isotope(s) in the alkane sulfonic acid are provided by using D₂SO₄ and T₂SO₄ respectively as solvent of the reaction and/or to provide SO₃ as oleum and/or
- the C isotope(s) in the alkane sulfonic acid is provided by using the corresponding C-labelled alkane as educt and/or
- the oxygen isotope(s) are provided by using the correspondingly labelled SO₃ as well as the correspondingly labelled H₂SO₄ as educt and/or solvent,
and/or
- the sulfur isotope(s) are provided by using the correspondingly labelled SO₃ as well as the correspondingly labelled H₂SO₄ as educt and/or solvent.

2. Method according to claim 1, wherein the alkane sulfonic acid is methane sulfonic acid.

3. Method according to claim 1 or 2, wherein the alkane sulfonic acid is labelled with ²H and/or ³H.

4. Method according to any of claims 1 to 3, wherein the alkane sulfonic acid is CD₃SO₃H, CHD₂SO₃H, or CH₂DSO₃H as well as mixtures of two or all the three of them and/or the alkane sulfonic acid is CT₃SO₃H, CHT₂SO₃H, or CH₂TSO₃H as well as mixtures of two or all the three of them.

5. Method according to any of claims 1 to 4, comprising the following steps:
a. Providing sulfur trioxide SO₃;
b. Providing an alkane, especially methane;
c. Providing a solvent,
d. Bringing into contact SO₃, alkane and the solvent in a high-pressure autoclave or laboratory reactor;
e. Setting a pressure of from 1 to 200 bar;
f. Adding an initiator or initiator precursor which is able to initialize the reaction between SO₃ and alkane at the described reaction conditions;
g. Controlling the temperature of the reaction mixture at 0 °C to 100 °C;
Letting react the compounds so that the alkane sulfonic acid, especially methane sulfonic acid, is formed;
**characterized in that**
the obtained alkane sulfonic acid is labelled with at least one isotope selected from ²H (D), ³H (T), ¹¹C, ¹³C,¹⁴C, ¹⁷O, ¹⁸O, ³³S, ³⁴S,³⁵S, ³⁶S,³⁸S as well of mixtures of two or more of them, wherein the concentration of the isotope is at least twice the amount of the natural amount of the respective isotope,
wherein
- the hydrogen isotope(s) in the alkane sulfonic acid are provided by using D₂SO₄ and T₂SO₄ respectively as solvent of the reaction and/or to provide SO₃ as oleum and/or
- the C isotope(s) in the alkane sulfonic acid is provided by using the corresponding C-labelled alkane as educt and/or
- the oxygen isotope(s) are provided by using the correspondingly labelled SO₃ as well as the correspondingly labelled H₂SO₄ as educt and/or solvent, and/or
- the sulfur isotope(s) are provided by using the correspondingly labelled SO₃ as well as the correspondingly labelled H₂SO₄ as educt and/or solvent.

6. Method according to claim 5, wherein the initiator or initiator precursor added at step f. is a compound selected from the group consisting of organic and inorganic peroxides being stable at room temperature, compounds with a heterolytically or homogenously cleavable bond, as well as mixtures of two or more of them.

7. Method according to claim 6, wherein the initiator or initiator precursor is ALK-SO₂-O-O-X, wherein ALK is a branched or unbranched alkyl group, especially a methyl, ethyl, propyl, butyl, isopropyl, isobutyl group, or a higher alkyl group, and X = hydrogen, zinc, aluminium, an alkali or alkaline earth metal.

## Patentansprüche

1. Verfahren zur Herstellung einer isotopenmarkierten Alkansulfonsäure, insbesondere Methansulfonsäure, umfassend die folgenden Schritte:
i) Bereitstellen eines Initiatorvorläufers,
ii) Bereitstellen von SO₃ und
iii) Bereitstellen des Alkans,
iv) Umsetzen des Initiatorvorläufers, SO₃ und des Alkans in einem Reaktor unter einem Druck von 1 bar bis zu 200 bar, wobei die Temperatur so gesteuert wird, dass sie in einem Bereich von 0°C bis 100°C liegt,
v) Abtrennen der so erhaltenen markierten Alkansulfonsäure von nicht umgesetzten Edukten und/oder gegebenenfalls Nebenprodukten,
**dadurch gekennzeichnet, dass**
die erhaltene Alkansulfonsäure mit wenigstens einem Isotop markiert ist, das aus ²H (D), ³H (T), ¹¹C, ¹³C, ¹⁴C, ¹⁷O ¹⁸O, ³³S, ³⁴S, ³⁵S, ³⁶S, ³⁸S sowie Gemischen von zweien oder mehreren davon ausgewählt ist, wobei die Konzentration des Isotops wenigstens doppelt so groß ist wie die natürliche Menge des jeweiligen Isotops,
wobei
- das oder die Wasserstoffisotope in der Alkansulfonsäure dadurch bereitgestellt werden, dass man D₂SO₄ bzw. T₂SO₄ als Lösungsmittel der Reaktion verwendet und/oder, um SO₃ als Oleum bereitzustellen, und/oder
- das oder die C-Isotope in der Alkansulfonsäure dadurch bereitgestellt werden, dass man ein entsprechendes C-markiertes Alkan als Edukt verwendet, und/oder
- das oder die Sauerstoffisotope dadurch bereitgestellt werden, dass man das entsprechend markierte SO₃ sowie die entsprechend markierte H₂SO₄ als Edukt und/oder als Lösungsmittel verwendet, und/oder
- das oder die Schwefelisotope dadurch bereitgestellt werden, dass man das entsprechend markierte SO₃ sowie die entsprechend markierte H₂SO₄ als Edukt und/oder als Lösungsmittel verwendet.

2. Verfahren gemäß Anspruch 1, wobei es sich bei der Alkansulfonsäure um Methansulfonsäure handelt.

3. Verfahren gemäß Anspruch 1 oder 2, wobei die Alkansulfonsäure mit ²H und/oder ³H markiert ist.

4. Verfahren gemäß einem der Ansprüche 1 bis 3, wobei es sich bei der Alkansulfonsäure um CD₃SO₃H, CHD₂SO₃H oder CH₂DSO₃H sowie Gemische von zweien oder allen dreien davon handelt und/oder es sich bei der Alkansulfonsäure um CT₃SO₃H, CHT₂SO₃H oder CH₂TSO₃H sowie Gemische von zweien oder allen dreien davon handelt.

5. Verfahren gemäß einem der Ansprüche 1 bis 4, umfassend die folgenden Schritte:
a. Bereitstellen von Schwefeltrioxid SO₃;
b. Bereitstellen eines Alkans, insbesondere Methan;
c. Bereitstellen eines Lösungsmittels;
d. In-Kontakt-Bringen von SO₃, Alkan und dem Lösungsmittel in einem Hochdruckautoklaven oder Laborreaktor;
e. Einstellen eines Drucks von 1 bis 200 bar;
f. Hinzufügen eines Initiators oder eines Initiatorvorläufers, der die Reaktion zwischen SO₃ und dem Alkan unter den beschriebenen Reaktionsbedingungen einleiten kann;
g. Regulieren der Temperatur des Reaktionsgemischs auf 0°C bis 100°C;
Reagierenlassen der Verbindungen, so dass die Alkansulfonsäure, insbesondere Methansulfonsäure, entsteht;
**dadurch gekennzeichnet, dass**
die erhaltene Alkansulfonsäure mit wenigstens einem Isotop markiert ist, das aus ²H (D), ³H (T), ¹¹C, ¹³C, ¹⁴C, ¹⁷O, ¹⁸O, ³³S, ³⁴S, ³⁵S, ³⁶S, ³⁸S sowie Gemischen von zweien oder mehreren davon ausgewählt ist, wobei die Konzentration des Isotops wenigstens doppelt so groß ist wie die natürliche Menge des jeweiligen Isotops,
wobei
- das oder die Wasserstoffisotope in der Alkansulfonsäure dadurch bereitgestellt werden, dass man D₂SO₄ bzw. T₂SO₄ als Lösungsmittel der Reaktion verwendet und/oder, um SO₃ als Oleum bereitzustellen, und/oder
- das oder die C-Isotope in der Alkansulfonsäure dadurch bereitgestellt werden, dass man ein entsprechendes C-markiertes Alkan als Edukt verwendet, und/oder
- das oder die Sauerstoffisotope dadurch bereitgestellt werden, dass man das entsprechend markierte SO₃ sowie die entsprechend markierte H₂SO₄ als Edukt und/oder als Lösungsmittel verwendet, und/oder
- das oder die Schwefelisotope dadurch bereitgestellt werden, dass man das entsprechend markierte SO₃ sowie die entsprechend markierte H₂SO₄ als Edukt und/oder als Lösungsmittel verwendet.

6. Verfahren gemäß Anspruch 5, wobei der in Schritt f. hinzugefügte Initiator oder Initiatorvorläufer eine Verbindung ist, die aus der Gruppe ausgewählt ist, die aus organischen und anorganischen Peroxiden, die bei Raumtemperatur stabil sind, Verbindungen mit einer heterolytisch oder homogen spaltbaren Bindung sowie Gemischen aus zweien oder mehreren davon besteht.

7. Verfahren gemäß Anspruch 6, wobei es sich bei dem Initiator oder Initiatorvorläufer um ALK-SO₂-O-O-X handelt, wobei ALK eine verzweigte oder unverzweigte Alkylgruppe, insbesondere eine Methyl-, Ethyl-, Propyl-, Butyl-, Isopropyl-, Isobutylgruppe oder eine höhere Alkylgruppe, ist und X = Wasserstoff, Zink, Aluminium, ein Alkali- oder Erdalkalimetall ist.

## Revendications

1. Procédé pour préparer un acide alcanesulfonique, notamment l'acide méthanesulfonique, marqué par isotopes, comprenant les étapes suivantes :
i) procurer un précurseur d'initiateur,
ii) procurer du SO₃, et
iii) procurer l'alcane,
iv) faire réagir le précurseur d'initiateur, le SO₃ et l'alcane dans un réacteur sous une pression de 1 bar jusqu'à 200 bar, en régulant la température dans la gamme allant de 0°C à 100°C,
v) séparer l'acide alcanesulfonique marqué ainsi obtenu de produits de départ n'ayant pas réagi et/ou éventuellement de sous-produits,
**caractérisé en ce que**
l'acide alcanesulfonique obtenu est marqué avec au moins un isotope choisi parmi ²H (D), ³H (T), ¹¹C, ¹³C, ¹⁴C, ¹⁷O, ¹⁸O, ³³S, ³⁴S, ³⁵S, ³⁶S, ³⁸S, et des mélanges de deux ou plus de ceux-ci, dans lequel la concentration de l'isotope est au moins deux fois la quantité naturelle de l'isotope respectif,
dans lequel
- le ou les isotopes d'hydrogène dans l'acide alcanesulfonique sont procurés en utilisant du D₂SO₄ et du T₂SO₄ respectivement comme solvant de la réaction, et/ou pour procurer du SO3 sous la forme d'oléum, et/ou
- le ou les isotopes de C dans l'acide alcanesulfonique sont procurés en utilisant l'alcane C-marqué correspondant comme produit de départ, et/ou
- le ou les isotopes d'oxygène sont procurés en utilisant le SO₃ marqué en conséquence, et le H₂SO₄ marqué en conséquence comme produit de départ et/ou solvant, et/ou
- le ou les isotopes de soufre sont procurés en utilisant le SO₃ marqué en conséquence, et le H₂SO₄ marqué en conséquence comme produit de départ et/ou solvant.

2. Procédé selon la revendication 1, dans lequel ledit acide alcanesulfonique est l'acide méthanesulfonique.

3. Procédé selon la revendication 1 ou 2, dans lequel ledit acide alcanesulfonique est marqué avec du ²H et/ou ³H.

4. Procédé selon l'une quelconque des revendications 1 à 3, dans lequel ledit acide alcanesulfonique est CD₃SO₃H, CHD₂SO₃H, ou CH₂DSO₃H ou de mélanges de deux ou tous les trois de ceux-ci, et/ou ledit acide alcanesulfonique est CT₃SO₃H, CHT₂SO₃H, ou CH₂TSO₃H ou de mélanges de deux ou tous les trois de ceux-ci.

5. Procédé selon l'une quelconque des revendications 1 à 4, comprenant les étapes suivantes consistant à :
a. procurer du trioxyde de soufre SO₃,
b. procurer un alcane, notamment le méthane,
c. procurer un solvant,
d. mettre le SO₃, l'alcane et le solvant en contact dans un autoclave à haute pression ou dans un réacteur de laboratoire,
e. régler une pression de 1 à 200 bar,
f. ajouter un initiateur ou précurseur d'initiateur qui peut initier la réaction entre le SO₃ et l'alcane dans les conditions de réaction décrites,
g. régler la température du mélange réactionnel à 0°C à 100°C,
faire réagir les composés de manière que l'acide alcanesulfonique, notamment l'acide méthanesulfonique, soit formé,
**caractérisé en ce que**
l'acide alcanesulfonique obtenu est marqué avec au moins un isotope choisi parmi ²H (D), ³H (T), ¹¹C, ¹³C, ¹⁴C, ¹⁷O, ¹⁸O, ³³S, ³⁴S, ³⁵S, ³⁶S, ³⁸S, et des mélanges de deux ou plus de ceux-ci, dans lequel la concentration de l'isotope est au moins deux fois la quantité naturelle de l'isotope respectif,
dans lequel
- le ou les isotopes d'hydrogène dans l'acide alcanesulfonique sont procurés en utilisant du D₂SO₄ et du T₂SO₄ respectivement comme solvant de la réaction, et/ou pour procurer du SO3 sous la forme d'oléum, et/ou
- le ou les isotopes de C dans l'acide alcanesulfonique sont procurés en utilisant l'alcane C-marqué correspondant comme produit de départ, et/ou
- le ou les isotopes d'oxygène sont procurés en utilisant le SO₃ marqué en conséquence, et le H₂SO₄ marqué en conséquence comme produit de départ et/ou solvant, et/ou
- le ou les isotopes de soufre sont procurés en utilisant le SO₃ marqué en conséquence, et le H₂SO₄ marqué en conséquence comme produit de départ et/ou solvant.

6. Procédé selon la revendication 5, dans lequel ledit initiateur ou précurseur d'initiateur ajouté à l'étape f. est un composé choisi dans le groupe consistant en peroxydes organiques et inorganiques qui sont stables à la température ambiante, des composés avec une liaison pouvant être clivée hétérolytiquement ou homogènement, ainsi que des mélanges de deux ou plusieurs de ceux-ci.

7. Procédé selon la revendication 6, dans lequel ledit initiateur ou précurseur d'initiateur est ALK-SO₂-O-O-X, où ALK est un groupe alkyle ramifié ou non ramifié, notamment un groupe méthyle, éthyle, propyle, butyle, isopropyle, isobutyle, ou un groupe alkyle supérieur, et X = hydrogène, zinc, aluminium, un métal alcalin ou alcalino-terreux.
